Europäisches Patentamt

(19) European Patent Office

Office européen des brevets

(11) **EP 0 539 319 B1**

(12) **EUROPEAN PATENT SPECIFICATION**

(45) Date of publication and mention
of the grant of the patent:
**08.12.1999 Bulletin 1999/49**

(51) Int. Cl.$^6$: **A61K 38/13**, A61K 47/14,
A61K 47/44

(21) Application number: **92810479.3**

(22) Date of filing: **23.06.1992**

(54) **Pharmaceutical cyclosporin composition**

Pharmazeutische Cyclosporin-Zusammensetzung

Composition pharmaceutique de cyclosphorine

(84) Designated Contracting States:
**BE DK ES GR NL PT SE**

(30) Priority: **27.06.1991 GB 9113872**

(43) Date of publication of application:
**28.04.1993 Bulletin 1993/17**

(60) Divisional application:
**99106122.7 / 0 953 630**

(73) Proprietor: **Novartis AG**
**4058 Basel (CH)**

(72) Inventors:
• **Hauer, Birgit**
**W-7630 Lahr (DE)**
• **Meinzer, Armin**
**W-7800 Freiburg-Munzingen (DE)**
• **Posanski, Ulrich**
**W-7800 Freiburg (DE)**
• **Vonderscher, Jacky**
**F-68400 Riedisheim (FR)**

(56) References cited:
CH-A- 641 356        FR-A- 2 636 534
FR-A- 2 642 650        GB-A- 2 218 334
GB-A- 2 228 198

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The present invention relates to novel galenic formulations, in particular novel galenic formulations in which the active ingredient comprises one or more members selected from cyclic poly-N-methylated undecapeptides of the cyclosporin class - see e.g. GB patent publications nos. 2 222 770 A and 2 228 198 A and equivalents world-wide.

As discussed in the said GB patent publications, the cyclosporins present highly specific difficulties in relation to administration generally and galenic formulation in particular, including in particular problems of drug bioavailability and variability in patient dose response.

In order to meet these and related difficulties, in GB patent publication no. 2 222 770 A, galenic formulations are disclosed comprising a cyclosporin as active ingredient and which take the form of, inter alia, a microemulsion or microemulsion pre-concentrate. Such compositions typically comprise 1) a hydrophilic phase, 2) a lipophilic phase and 3) a surfactant. Specifically recited hydrophilic phase components are the products known and commercially available under the trade names Transcutol and Glycofurol as well as 1,2-propylene glycol. Preferred lipophilic phase components are medium chain fatty acid triglycerides such as known and commercially available under the trade names Miglyol, Captex, Myritol, Capmul, Captex, Neobee and Mazol, Miglyol 812 being the most preferred. Suitable surfactant components include, in particular, reaction products of natural or hydrogenated vegetable oils and ethylene glycol such as those known and commercially available under the trade names Cremophor and Nikkol, the products Cremophor RH40 and Nikkol HCO-40 being indicated as especially preferred.

GB patent publication no. 2 228 198 A proposes an alternative means for meeting difficulties in relation to cyclosporin administration. Specifically it discloses oil based formulations in which the oily component comprises a combination of tri-glyceride and (i) glycerol partial esters or (ii) 1,2-propylene glycol complete or partial esters or (iii) sorbitol complete or partial esters. The products known and commercially available under the tradename Maisine are proposed as suitable tri- and partial glyceride components. The disclosed compositions additionally comprise a surfactant component, for example Cremophor RH40, but are preferably free of any hydrophilic components such as ethanol. Described and exemplified compositions are free of such components.

In accordance with the present invention it has now surprisingly been found that particularly stable cyclosporin galenic formulations having particularly interesting bioavailability characteristics and reduced variability in inter- and intra-subject bioavailability parameters, are obtainable. Such compositions being new, the present invention provides in its broadest aspect:

A pharmaceutical composition comprising a cyclosporin as active ingredient in a carrier medium comprising:

    1) 1,2-propylene glycol;
    2) a mixed $C_{12}$-$C_{20}$ fatty acid mono-, di-, tri-glyceride, and
    3) a surfactant,

which composition is a microemulsion preconcentrate.

The term "pharmaceutical composition" as used herein and in the accompanying claims is to be understood as defining compositions of which the individual components or ingredients are themselves pharmaceutically acceptable, e.g. where oral administration is foreseen, suitable or acceptable for oral application.

Cyclosporins to which the present invention applies are any of those having pharmaceutical utility, e.g. as immunosuppressive agents, anti-parasitic agents and agents for the reversal of multi-drug resistance, as known and described in the art, in particular Cyclosporin A (also known as and referred to hereinafter as Ciclosporin), Cyclosporin G, [O-(2-hydroxyethyl)-(D)Ser]$^8$-Ciclosporin, and [3'-deshydroxy-3'-keto-MeBmt]$^1$-[Val]$^2$-Ciclosporin.

Components (2) in the compositions of the invention comprise mixtures of $C_{12-20}$ fatty acid mono-, di- and tri-glycerides, especially $C_{16-18}$ fatty acid mono-, di- and triglycerides. The fatty acid component of said mixed mono-, di- and tri-glycerides may comprise both saturated and unsaturated fatty acid residues.

Preferably however they will predominantly be comprised of unsaturated fatty acid residues in particular, $C_{18}$ unsaturated fatty acid residues for example linolenic, linoleic and oleic acid residues. Suitably component (2) will comprise at least 60%, preferably at least 75%, more preferably 85% or more by weight $C_{18}$ unsaturated fatty acid, e.g. linolenic, linoleic and oleic acid mono-, di- and tri-glycerides. Suitably they will comprise less than 20%, e.g. ca. 15% or 10% by weight or less, saturated fatty acid, e.g. palmitic and stearic acid mono-, di- and tri-glycerides.

Components (2) in the compositions of the invention will preferably be predominantly comprised of mono- and di-glycerides, e.g. comprise at least 50%, more preferably at least 70%, e.g. 75%, 80%, 85% by weight or more, mono- and di-glycerides, based on the total weight of component (2).

Components (2) in the compositions of the invention will suitably comprise from about 25 to about 50%, preferably from about 30 to about 40%, e.g. 35 to 40%, monoglycerides, based on the total weight of component (2).

Components (2) in the composition of the invention will suitably comprise from about 30 to about 60%, preferably from about 40 to about 55%, e.g. about 48 to 50%, di-glycerides, based on the total weight of component (2).

Components (2) in the compositions of the invention will suitably comprise at least 5%, e.g. from about 7.5 to about 15%, e.g. 9 to 12%, by weight of triglycerides.

Components (2) in the compositions of the invention may be prepared by admixture of individual mono-, di- or tri-glycerides in appropriate relative proportion. Conveniently however they will comprise transesterification products of vegetable oils, for example almond oil, ground nut oil, olive oil, peach oil, palm oil or, preferably, corn oil, sunflower oil or safflower oil and most preferably corn oil, with glycerol.

Such transesterification products are generally obtained by heating of the selected vegetable oil with glycerol, at high temperature in the presence of an appropriate catalyst under an inert atmosphere with continuous agitation, e.g. in a stainless steel reactor, to effect trans-esterification or glycerolysis. In addition to their mono-, di-and tri-glyceride components, such transesterification products will also generally comprise minor amounts of free glycerol. The amount of free glycerol present in components (2) for use in the compositions of the invention will preferably be less than 10%, more preferably less than 5%, most preferably ca. 1 or 2% by weight based on the total weight of free glycerol plus mono-, di- and tri-glycerides.

Preferably some of the glycerol is first removed e.g. by distillation (to give a "substantially glycerol free batch"), when soft gelatine capsules are to be made.

Especially suitable components (2) for use in the compositions of the invention will thus comprise the following components in the indicated amounts by weight based on the total weight of component (2):

Mono-glycerides: 25 or 30 to 50%, especially 30 to 40%.
Di-glycerides: 30 or 40 to 60%, especially 40 to 55%, e.g. 45 to 55%.
Mono- plus di-glycerides: >75%, especially >80%, e.g. ca. 85%.
Tri-glycerides: at least 5%.
Free glycerol: <5%, preferably <2% or <1%.

Particularly suitable components (2) for use in the compositions of the invention are trans-esterification products of corn oil and glycerol, for example as commercially available under the trade name Maisine. Such products are comprised predominantly of linoleic and oleic acid mono-, di- and tri-glycerides together with minor amounts of palmitic and stearic acid mono-, di- and tri-glycerides (corn oil itself being comprised of ca. 56% by weight linoleic acid, 30% oleic acid, ca. 10% palmitic and ca. 3% stearic acid constituents). Physical characteristics for Maisine [available from the company Etablissements Gattefossé, of 36, Chemin de Genas, P.O.Box 603, 69804 Saint-Priest, Cedex (France)] are: approximate composition

| | |
|---|---|
| free glycerol | - 10% max. (typically 3.9-4.9% or, in "substantially glycerol free" batches, ca. 0.2%) |
| mono-glycerides | - ca. 35% (typically 30-40% or, in "substantially glycerol free" batches, e.g. 32-36%, e.g. ca. 36%) |
| di-glycerides | - ca. 50% (or, in "substantially glycerol free" batches ca. 46-48%) |
| tri-glycerides | - ca. 10% (or, in "substantially glycerol free" batches, ca. 12-15%) |
| free oleic acid content | - ca. 1% |

Further physical characteristics for Maisine are: acid value = max. ca. 2, iodine no. = ca. 85-105, saponification no. = ca. 150-175 (Fiedler "Lexikon der Hilfsstoffe", 3rd revised and expanded edition (1989) Vol. 2, p.768). The fatty acid content for Maisine is typically: palmitic acid - ca. 11%; stearic acid - ca. 2.5%; oleic acid - ca. 29%; linoleic acid - ca. 56%; others - ca. 1.5%.

It is especially preferred that the component (2) e.g. a glycerol transesterified corn oil is clear, e.g. after keeping a sample in a refrigerator, e.g. between 2 and 8°C, for 24 hours, the sample is clear at room temperature 1 hour after taking the sample out of the refrigerator.

Preferably components (2) have a low saturated fatty acid content. Components (2) meeting these requirements may, for example be obtained from commercially available products, e.g. obtained therefrom by methods such as separative techniques as known in the art, e.g. freezing procedures coupled with separative techniques, e.g. centrifugation, to remove the saturated fatty acid components/enhance the unsaturated fatty acid component content. Typically the total saturated fatty acid component content will be <15%, e.g. <10%, or <5%by weight based on the total weight of component (2). A reduction of the content of saturated fatty acid component in the mono-glyceride fraction of components (2) may be observed after the separative technique.

Components (2) thus preferably contain lesser quantities of saturated fatty acids (e.g. palmitic and stearic acids) and relatively greater quantities of unsaturated fatty acids (e.g. oleic and linoleic acids) than for the starting material.

Typical preferred components (2) may according to the preferred embodiment of this invention contain:

32-36% mono-glycerides,
45-55% di-glycerides and
12-20% tri-glycerides

by weight based on the total weight of component (2).

Further preferred characteristics include the following:

Fatty acid content as determined as the methyl ester by chromatography

| | |
|---|---|
| Methyl linoleate | 53-63% |
| Methyl oleate | 24-34% |
| Methyl linolenate | 0- 3% |
| Methyl arachate | 0- 3% |
| Methyl palmitate | 6-12% |
| Methyl stearate | 1- 3% |
| | |
| Relative Density | 0.94-0.96 |
| Hydroxyl Value | 140-210 |
| Iodine Value | 110-120 |
| Peroxide Value | <4.0 |
| Free Glycerol | <1.0 |
| Acid value max. | ca. 2 |
| Saponification no. ca. | 150-185 |

Components (2) complying with the above outlined features are referred to hereafter as "refined glycerol-transesterified corn oils". Freshly prepared components (2) according to the preferred embodiments are of clear appearance and stay clear at storage temperature of 20°C - 25°C for more than 20 days.

The "refined glycerol-transesterified corn oils" have especially been proposed for the preparation of the compositions of this invention. They may also have uses for the solubilization of other active agents and have the advantage of remaining stable, e.g. clear, for a long time.

Components (3) in the compositions of the invention preferably have an HLB of at least 10.

Examples of suitable components (3) in the compositions of the invention are:

3.1 Reaction products of a natural or hydrogenated castor oil and ethylene oxide. Such products may be obtained in known manner, e.g. by reaction of a natural or hydrogenated castor oil with ethylene oxide, e.g. in a molar ratio of from about 1:35 to about 1:60, with optional removal of the polyethyleneglycol component from the product, e.g. in accordance with the methods disclosed in German Auslegeschriften 1,182,388 and 1,518,819. Especially suitable are the various tensides available under the trade name Cremophor. Particularly suitable are the products Cremophor RH 40 having a saponification number of ca. 50-60, an acid number <1, an iodine number <1, a water content (Fischer) <2%, an $n_D^{60}$ of ca. 1,453 - 1,457 and an HLB of ca. 14 - 16; Cremophor RH 60 having a saponification number of ca. 40 - 50, an acid number <1, an iodine number <1, a water content (Fischer) 4.5-5.5%, and an $n_D^{25}$ of ca. 1.453-1.457 and an HLB of ca. 15-17; and Cremophor EL having a molecular weight (by steam osmometry) of ca. 1630, a saponification number of ca. 65-70, an acid number of ca. 2, an iodine number of ca. 28-32 and an $n_D^{25}$ of ca. 1.471.(c.f. Fiedler, "Lexikon der Hilfstoffe", 3rd revised and expanded edition (1989), Vol.1,p.326). Also suitable for use in this category are the various tensides available under the trade name Nikkol (e.g. Nikkol HCO-40 and HCO-60), Emulgin (e.g. Emulgin RO40), Mapeg (e.g. Mapeg CO-40h) and Incrocas (e.g. Incrocas 40) (c.f. Fiedler). The said product Nikkol HCO-60 is a reaction product of hydrogenated castor oil and ethylene oxide exhibiting the following characteristics: acid value ca. 0.3; saponification number of ca. 47.4; hydroxy value of ca. 42.5; pH (5%) of ca. 4.6; color APHA = ca. 40; m.p. = ca. 36.0°C; freezing point = ca. 32.4°C; $H_2O$ content (%, KF) = 0.03.

3.2 Polyoxyethylene-sorbitan-fatty acid esters, e.g. mono- and tri-lauryl, palmityl, stearyl and oleyl esters, e.g. of the type known and commercially available under the trade name Tween (c.f. Fiedler, loc.cit. p.1300-1304) including the products Tween

> 20 [polyoxyethylene(20)sorbitanmonolaurate],
> 21 [polyoxyethylene(4)sorbitanmonolaurate],
> 40 [polyoxyethylene(20)sorbitanmonopalmitate],
> 60 [polyoxyethylene(20)sorbitanmonostearate],
> 65 [polyoxyethylene(20)sorbitantristearate],
> 80 [polyoxyethylene(20)sorbitanmonooleate],
> 81 [polyoxyethylene(5)sorbitanmonooleate],
> 85 [polyoxyethylene(20)sorbitantrioleate].

Especially preferred products of this class for use in the compositions of the invention are the above products Tween 40 and Tween 80.

3.3 Polyoxyethylene fatty acid esters, for example polyoxyethylene stearic acid esters of the type known and commercially available under the trade name Myrj (c.f. Fiedler, loc. cit., 2, p.834-835); an especially preferred product of this class for use in the compositions of the invention is the product Myrj 52 having a $D^{25}$= ca. 1.1., m.p. = ca. 40-44°C, an HLB value = ca. 16.9., an acid value = ca. 0-1 and a saponification no. = ca. 25-35.

3.4 Polyoxyethylene-polyoxypropylene co-polymers and block co-polymers, e.g. of the type known and commercially available under the trade names Pluronic, Emkalyx and Poloxamer (c.f. Fiedler, loc. cit., 2, p. 959). An especially preferred product of this class for use in the compositions of the invention is the product Pluronic F68, having an m.p. = ca. 52°C and a molecular weight of ca. 6800-8975. A further preferred product of this class for use in the compositions of the invention is the product Poloxamer 188.

3.5 Dioctylsulfosuccinate or di-[2-ethylhexyl]-succinate (c.f. Fiedler, loc. cit., 1, p. 107-108).

3.6 Phospholipids, in particular lecithins (c.f. Fiedler, loc. cit., 2, p. 943-944). Lecithins suitable for use in the compositions of the invention include, in particular, soya bean lecithins.

3.7 Propylene glycol mono- and di-fatty acid esters such as propylene glycol dicaprylate (also known and commercially available under the trade name Miglyol 840), propylene glycol dilaurate, propylene glycol hydroxystearate, propylene glycol isostearate, propylene glycol laurate, propylene glycol ricinoleate, propylene glycol stearate and so forth (c.f. Fiedler, loc. cit., 2, p. 808-809).

3.8 Sodium lauryl sulfate.

For use in relation to the present invention, components as set out under (3.1) above are most preferred.

Components (1), (2) and (3) are present in the compositions of the invention in relative proportions such that the composition is a "micro-emulsion preconcentrate", i.e. having the characteristics of a micro-emulsion preconcentrate system as described in GB patent publication no. 2 222 770 A at pages 11 to 12, the contents of which are, for the purposes of defining such systems, incorporated herein by reference. Compositions of the invention are thus "microemulsion preconcentrates", in particular of the type providing o/w (oil-in-water) microemulsions. The present invention is also to be understood as including compositions comprising components (1), (2) and (3) together with (4) water and which are microemulsions.

As also indicated in GB patent publication no. 2 222 770 A the hydrophilic phase of microemulsion preconcentrate systems, i.e. component (1) in compositions of the present invention may include one or more additional ingredients as hydrophilic phase component, for example lower (e.g. $C_{1-5}$) alkanols, in particular ethanol. Such components will generally be present in partial replacement of component (1). While the use of ethanol in compositions of the present invention is not essential, it has been found to be of particular advantage when the compositions are to be manufactured in soft gelatine encapsulated form, e.g. as a means of improving storage characteristics, in particular to reduce risk of cyclosporin precipitation following encapsulation procedures. Thus the shelf life stability may be extended by employing a lower alkanol as an additional ingredient of the hydrophilic phase.

Suitably the hydrophilic phase component, i.e. component (1), 1,2-propylene glycol, or component (1) plus any hydrophilic phase co-component(s), e.g. ethanol, will be present in the compositions of the invention in an amount of from 1.0 or 2.5 to 50%, preferably from 5 to 40%, more preferably from 10 to 35%, e.g. above 15%, e.g. from about 20 to about 30% by weight based on the total weight of hydrophilic phase component(s) plus components (2) and (3).

When a hydrophilic phase co-component is employed, the co-component, e.g. ethanol, is suitably present in an amount of up to about 20%, preferably up to about 10 or 15%, e.g. from about 5 to 10 or 15% by weight based in the total weight of the composition. Such co-component is thus suitably present in an amount of from about 25 to 75% by weight based on the total weight of hydrophilic phase components (e.g. 1,2-propylene glycol plus ethanol) more preferably it is present in an amount of less than 50%, e.g. from 25 to 50%, for example about 30, 40 or 50%.

Suitably component (2) will be present in the compositions of the invention in an amount of from 5 to 65%, preferably from 15 to 45%, more preferably from 20 to 40%, e.g. from about 25 to about 35%, based on the total weight of hydrophilic phase component(s) plus components (2) and (3).

Suitably component (3) will be present in the compositions of the invention in an amount of from 25 to 75%, preferably from 30 to 60%, e.g. from about 55 or 60% based on the total weight of hydrophilic phase component(s) plus components (2) and (3).

Suitably the compositions of the invention will comprise from about 1 or 2 to 30%, preferably from 5 to 20 or 25%, more preferably from 7.5 to 15%, e.g. about 10% by weight of cyclosporin based on the total weight of the composition.

Accompanying Figure I represents a three-way plot for relative concentrations of hydrophilic phase component, i.e. 1,2-propylene glycol, component (2), e.g. "refined glycerol-transesterified corn oil", and component (3), e.g. Cremophor RH40, in compositions in accordance with the invention and comprising 10% cyclosporin (e.g. Ciclosporin) by weight. Relative concentrations of the carrier components increase in the directions indicated by the arrows from 0 to 100%.

For compositions in accordance with the present invention the relative proportion of hydrophilic phase component(s), component (2) and component (3) will suitably lie within the shaded area X. Compositions thus defined are microemulsion preconcentrates of high stability, capable on addition to water, of providing microemulsions having an average particle size of <1,500Å and stable over periods in excess of 24 hrs. In contrast compositions in the region A, B and C give aqueous systems subject to (A) discoloration, (B) phase separation and (C) turbidity respectively. Compositions in accordance with the invention comprising hydrophilic phase component(s) and components (2) and (3) in relative proportion as defined by the line X of Fig. I are accordingly especially preferred.

In the event that the 1,2-propylene glycol component is partially replaced by ethanol as hereinbefore described, the area X of Fig. I is shifted slightly upwards within the plot, i.e. in the direction of higher component (3) concentration. This shift however represents an upwards displacement of a few percent only and does not substantially alter the obtained plot.

The compositions of the invention show good stability characteristics, e.g. as indicated by standard stability trials, e.g. having a shelf life stability of up to three years, and even longer.

Compositions in accordance with the present invention may also include further additives or ingredients, for example [e.g. antioxidants ascorbyl palmitate, butyl hydroxy anisole (BHA), butyl hydroxy toluene (BHT) and tocopherols, e.g. $\alpha$-tocopherol (vitamin E)] and/or preserving agents, e.g. in an amount of from about 0.05 to 1% by weight based on the total weight of the composition, or sweetening or flavoring agents, e.g. in an amount of up to about 2.5 or 5% by weight based on the total weight of the composition.

Compositions in accordance with the present invention have been found to exhibit especially advantageous properties when administered orally, e.g. in terms of both the consistency and high level of bioavailability achieved as indicated in standard bioavailability trials e.g. in healthy patients using a specific monoclonal kit to determine cyclosporin levels, e.g. as described in the Examples hereinafter. In particular the compositions in accordance with the present invention provide an improved oral administration form for cyclosporins (e.g. Ciclosporin) as it exhibits absence of significant food interaction, which we have observed with the commercially available oral form of Ciclosporin especially with fat rich food. Moreover, inter-subject and intra-subject variability of pharmacokinetic parameters may be significantly lower with the compositions according to the present invention than with the commercial oral form of Ciclosporin. Specifically the difference between the pharmacokinetic parameters with food intake and without food intake, or even between day time absorption and night time absorption, may be eliminated by administering the composition in accordance with the present invention. Thus with the novel composition according to present invention the pharmacokinetic parameters, e.g. absorption and blood levels, become surprisingly more predictable and this new galenic form may eliminate problems in administration with erratic absorption of Ciclosporin. Additionally the composition according to present invention, may exhibit an improved bioavailability in patients having malabsorption, e.g. liver transplantation patients or pediatric patients. In particular it has been found that such compositions are compatible with tenside materials, e.g bile salts, present in the gastro-intestinal tract. That is, they are fully dispersible in aqueous systems comprising such natural tensides and are thus capable of providing microemulsion systems in situ which are stable and do not exhibit precipitation of the cyclosporin or other disruption of fine particulate structure. Function of such systems on oral administration remains independent of and/or unimpaired by the relative presence or absence of bile salts at any particular time or for any given individual.

The compositions of the invention are well tolerated, e.g. as indicated by clinical trials over 4 weeks.

Compositions in accordance with the present invention will preferably be compounded in unit dosage form, e.g.

by filling into orally administerable capsule shells, e.g. soft or hard gelatine capsule shells but if desired may be in drink solution form. Where compositions are in unit dosage form, each unit dosage will suitably contain between 10 and 200 mg cyclosporin, more suitably between 10 and 150 mg, e.g. 15, 20, 25, 50 or 100 mg cyclosporin. Such unit dosage forms are suitable for administration 1x, 2x or 3x up to 5x daily (e.g. depending on the particular purpose of therapy, the phase of therapy etc.).

Alternatively compositions in accordance with the present invention suitable for oral administration may include (4) water or any other aqueous system, to provide microemulsion systems suitable for drinking.

In addition to the foregoing the present invention also provides a process for the production of a pharmaceutical composition as hereinbefore defined, which process comprises bringing a component (1), a component (2) and a component (3) as hereinbefore defined into intimate admixture and, when required compounding the obtained composition in unit dosage form, for example filing said composition into gelatine, e.g. soft or hard gelatine, capsules.

In a more particular embodiment the present invention provides a process for the production of a pharmaceutical composition as hereinbefore defined in the form of a "microemulsion preconcentrate" or microemulsion, which method comprises bringing a component (1), a component (2) and a component (3), optionally together with further components or additives, in particular with a hydrophilic phase co-component, for example ethanol, into intimate admixture in relative proportions of components (1), (2) and (3), such that a microemulsion preconcentrate is obtained and, when required, compounding the obtained composition in unit dosage form or combining said obtained composition with sufficient water or sufficient of an aqueous solvent medium such that a microemulsion is obtained.

The following examples are illustrative of compositions in accordance with the invention, in unit dosage form, suitable for use, e.g. in the prevention of transplant rejection or for the treatment of autoimmune disease, on administration of from 1 to 5 unit dosages/day. The examples are described with particular reference to Ciclosporin. However equivalent compositions may be obtained employing any other cyclosporin, in particular [O-(2-hydroxyethyl)-(D)-Ser]$^8$-Ciclosporin (hereinafter referred to as Compound Z).

## EXAMPLE 1:

Preparation of "refined glycerol-transesterified corn oil".

Substantially-glycerol free glycerol-transesterified corn oil (if necessary after heating to give a clear mixture) is slowly cooled to a temperature of +20°C and kept at this temperature for one night. In a first-step centrifugation, at an acceleration of 12 000 G and a flow rate of 103 kg/h in a continuous flow centrifuge, a liquid phase (62 kg/h) and a sediment-containing phase (41 kg/h) are obtained. The liquid phase is slowly cooled to +8°C and kept at this temperature for one night. In a second-step centrifugation at an acceleration of 12 000 G and a flow rate of 112 kg/h a liquid phase (76.2 kg/h) and a sediment-containing phase (35.8 kg/h) are obtained. The liquid phase is "refined glycerol-transesterified corn oil". Alternatively an improved product may be obtained by effecting the centrifugation in three steps, e.g. at +20°C, +10°C and +5°C.

The process is characterised by a slight percentage reduction in the mono-glyceride component in the refined glycerol transesterified corn oil as compared to the starting material (e.g. 35.6% compared to 38.3%).

A typical analytical comparison between the sediment and clear solution is as follows:

| Compound | Sediment (%) | Clear Solution (%) |
|---|---|---|
| 1. Mono palmitate | 19.1 | 3.4 |
| 2. Mono linoleate + Mono oleate | 23.4 | 27.0 |
| 3. Mono stearate | 5.7 | <2 |
| 4. Dilinoleate + Dioleate | 35.4 | 44.7 |
| 5. Other di-glycerides | 7.7 | 10.4 |
| 6. Tri-glycerides | 8.7 | 12.5 |

Typical contents of components in the refined product obtained from these preparations are listed in the following Table:

| COMPOSITION OF COMPONENTS (% w/w) | | |
|---|---|---|
| Components | | refined glycerol-trans-esterified corn oil |
| Glycerides: | mono | 33.3 |
| | di | 52.1 |
| | tri | 14.6 |
| Fatty acids: | | |
| palmitic acid (C16) | | 7.8 |
| stearic acid (C18) | | 1.7 |
| oleic acid (C18:1) | | 31.6 |
| linoleic acid (C18:2) | | 57.7 |
| Glycerol content | | <1% |

## EXAMPLE 2:

Preparation of oral unit dosage forms

| COMPONENT | QUANTITY (mg/capsule) |
|---|---|
| Cyclosporin, e.g. Ciclosporin | 100 |
| 1) 1,2-propylene glycol | 200 |
| 2) refined oil | 320 |
| 3) Cremophor RH40 | 380 |
| Total | 1,000 |

The cyclosporin is dissolved in (1) with stirring at room temperature and (2) and (3) are added to the obtained solution again with stirring. The obtained mixture is filled into size 1 hard gelatine capsules and sealed e.g. using the Quali-Seal technique.

Compositions comprising 50 and 100 mg Ciclosporin, are prepared analogously employing the following indicated ingredients in the indicated amounts.

In this Example, refined oil = "refined glycerol-transesterified corn oil" as described in Example 1 or Maisine, e.g. substantially glycerol free Maisine.

COMPOSITIONS COMPRISING 100 mg cyclosporin, e.g. Ciclosporin

| COMPOSITION | 2 | 3 | 4 | 5 | 6 |
|---|---|---|---|---|---|
| COMPONENT | QUANTITY (mg/capsule) | | | | |
| 1) 1,2-Propylene glycol | 200 | 270 | 180 | 180 | 90 |
| 2) refined oil | 350 | 180 | 180 | 360 | 360 |
| 3) Cremophor RH40 | 350 | 450 | 540 | 360 | 450 |

| COMPOSITION | 7 | 8 | 9 | 10 |
|---|---|---|---|---|
| COMPONENT | QUANTITY (mg/capsule) | | | |
| 1) 1,2-Propylene glycol | 150 | 100 | 200 | 200 |
| 1a) ethanol | 100 | 100 | 100 | 100 |
| 2) refined oil | 345 | 320 | 320 | 290 |
| 3) Cremophor RH40 | 405 | 380 | 380 | 360 |

COMPOSITIONS COMPRISING 50 mg Ciclosporin

| COMPOSITION | A | B | C | D | E | F |
|---|---|---|---|---|---|---|
| COMPONENT | QUANTITY (mg/capsule) | | | | | |
| 1) 1,2-Propylene glycol | 100 | 135 | 45 | 90 | 100 | 50 |
| 1a) ethanol | | | | | | 50 |
| 2) refined oil | 160 | 90 | 180 | 180 | 67 | 160 |
| 3) Cremophor RH40 | 190 | 225 | 225 | 180 | 167 | 190 |

As indicated above equivalent compositions may be made containing Compound Z instead of Ciclosporin. Thus composition D may be made containing 50 mg Compound Z instead of Ciclosporin.

**EXAMPLE 3: Bioavailability in dogs**

The biopharmaceutical properties of compositions in accordance with the present invention we compared with the marketed soft-gelatine capsule of Ciclosporin. The forms were compared after oral administration to 12 male beagle dogs in a cross-over design. The pharmacokinetic profile of Ciclosporin was determined in whole blood over 24 hours. the areas under the curve of the blood concentration versus time curves (AUC), $C_{max}$ and $T_{max}$ were determined.
Forms: Dose 100 mg Ciclosporin/dog

9

| Composition X (commercial form, soft gelatin capsule) | |
|---|---|
| Ciclosporin | 100 mg |
| Labrafil | 300 mg |
| ethanol | 100 mg |
| Maize oil | 416 mg |
| Total | 926 mg/dosage |

| Composition I according to present invention (a soft gelatin capsule): | |
|---|---|
| Ciclosporin | 100 mg |
| 1) 1, 2-propylene glycol | 75 mg |
| 1a) ethanol | 150 mg |
| 2) refined glycerol-transesterified corn oil | 345 mg |
| 3) Cremophor RH40 | 405 mg |
| Total | 1075 mg/dosage |

Drug administration:

10 male beagle dogs weighing around 12 kg completed the trial successfully. Twenty hours before the drug administration the food was withdrawn but the animals were allowed free access to water until the beginning of the experiment. The dosage forms were administered by gavage to the animals, early in the morning (approx. 8.00 am), and followed by 20 ml NaCl 0.9% solution. Three hours after the administration, the animals were again allowed free access to water and food. A 1 week wash-out period was necessary between 2 administrations to the same animal.

Blood sampling:

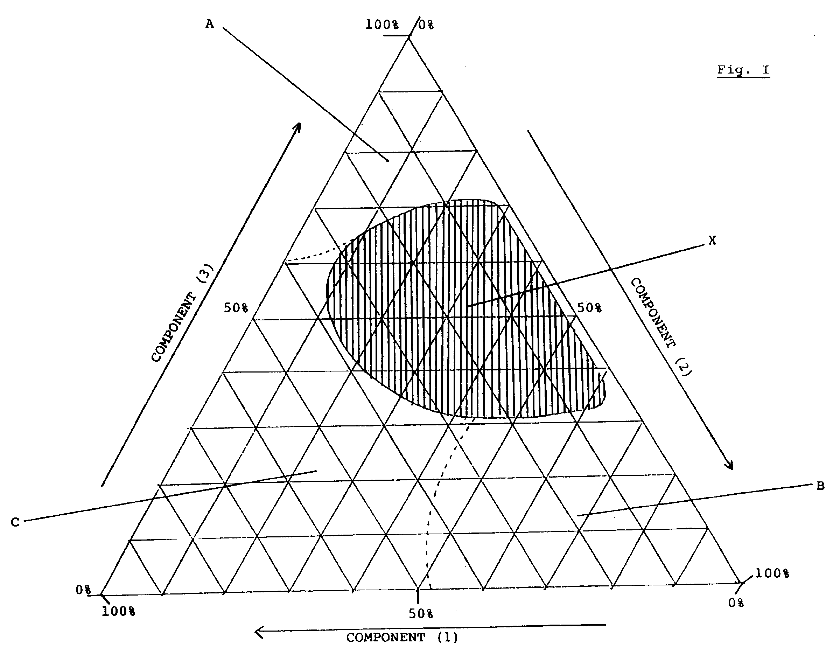

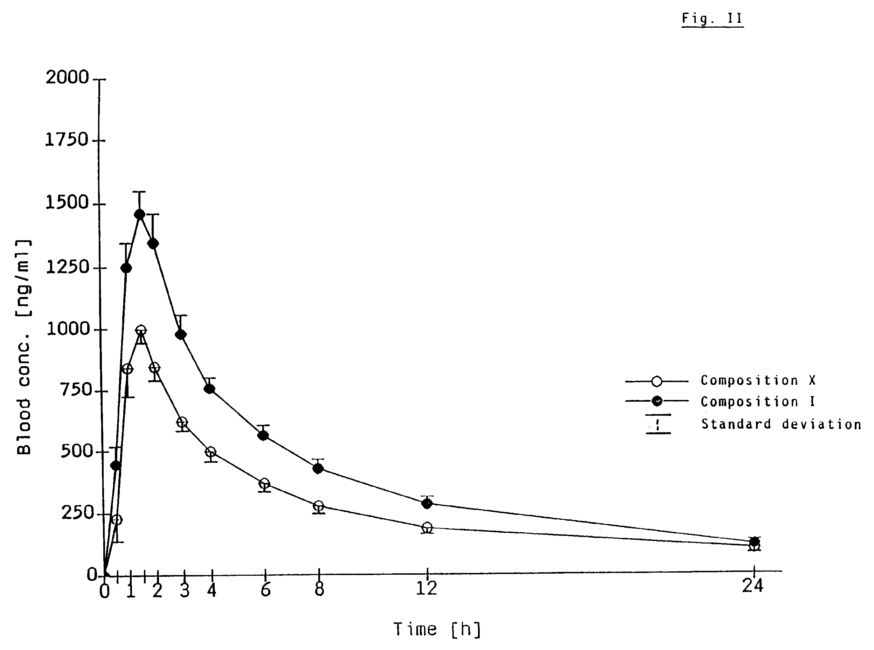

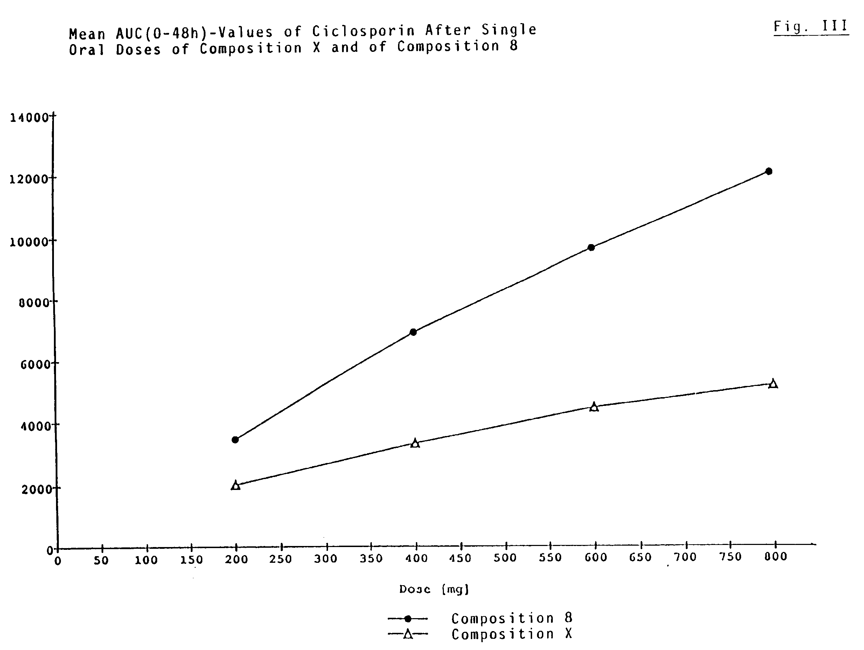

Blood samples of 2 ml (or 5 ml for the blank sample) were taken from the vena cephalica (forearm) with a sterile needle (diameter ca. 1.2 mm) and collected into 5 ml plastic tubes containing EDTA at -15 min, 30 min, 1, 1.5, 2, 3, 4, 6, 8, 12 and 24 hours after the oral administration of the drug. The blood samples were stored at ca. -18°C until drug assay. The blood samples were analysed by Ciclosporin-specific radioimmunoassay (RIA). The median blood concentrations of Ciclosporin in dogs are plotted in the accompanying Figure II. The areas under the blood drug concentration versus time curves (AUC) were calculated using the trapezoidal rule. An analysis (CV) of variance was performed and the mean AUCs, Cmax and Tmax were compared statistically by the Tukey test. The results obtained are shown in the following table.

| Composition | $AUC_{0-24h}$ | | $C_{max}$ | | $T_{max}$ | |
|---|---|---|---|---|---|---|
| | Mean [ng.h/ml] | CV [%] | Mean [ng/ml] | CV [%] | Mean [h] | CV [%] |
| X | 6695 | 27 | 1053 | 25 | 1.3 | 20 |
| I | 10064 | 24 | 1539 | 18 | 1.6 | 29 |

The behaviour and body weight of the animals were controlled during the study. No body weight loss could be detected.

Conclusion: The composition according to the present invention (composition I) has a significantly higher bioavailability (factor 1.5) than the commercial soft-gelatin capsule of Ciclosporin.

Figure II shows the average whole blood Ciclosporin concentrations as determined by a specific monoclonal RIA following single oral administration of Composition X and Composition I each in 100 mg dosage. Blood concentration (in ng/ml) is recorded vertically and time horizontally.

## EXAMPLE 4: Bioavailability in humans

The bioavailability of Ciclosporin is compared as it is determinable after administration of the commercial Ciclosporin soft gelatine capsule and of a composition according to present invention.

Administered form: 100 mg Ciclosporin per capsule

| Composition X (commercial form, soft gelatine capsule) | |
|---|---|
| Ciclosporin | 100 mg |
| Labrafil | 300 mg |
| Ethanol | 100 mg |
| Maize oil | 426 mg |
| Total | 926 mg/Capsule |

Composition No. 8 (according to Example 2 containing "refined glycerol-transesterified corn oil") in a soft gelatine capsule.

Method:

Forty eight healthy male subjects completed the study. Each of the participants received four of the eight administrations (two doses of composition 8 and the same two doses of composition X).

The participants were randomly allocated to two subgroups consisting of twenty four subjects each according to a parallel design. Subjects in Group I received doses of 200 mg and 600 mg Ciclosporin and subjects in Group II received 400 mg and 800 mg.

Within each of the two groups the trial was conducted on the basis of a balanced 4-way cross-over design with a wash-out period of two weeks between each treatment.

Blood samples for determination of Ciclosporin in whole blood were taken 1 minute before drug intake and then 15 min, 30 min. 45 min, 1 h, 1.5h, 2 h, 2.5 h, 3 h, 3.5 h, 4 h, 4.5 h, 5 h, 6 h, 8 h, 20 h, 12 h, 16 h, 20 h, 24 h, 28 h, 32 h, 36 h, 40 h, and 48 h after drug intake.

The individual concentrations of Ciclosporin in whole blood were determined for each blood sample by a specific RIA-method.

The limit of quantification was 12.5 ng/ml.

Blood concentrations and corresponding $AUC_{(0-48\ h)}$-values of Ciclosporin were significantly higher after administration of Composition 8 than after administration of Composition X at all dosage strengths. Peak concentrations ($C_{max}$) of the 200 mg, 400 mg, and 600 mg dose levels appeared somewhat earlier after administration of Composition 8 (see following table).

Table

| Bioavailability of Ciclosporin in Humans | | | |
|---|---|---|---|
| Mean (±SD) values of $AUC_{(0-48\ h)}$, $C_{max}$ and $T_{max}$ after single oral administration of different dosages of Composition X and Composition 8 | | | |
| Form | $AUC_{(0-48\ h)}$ [ng.h/ml] | $C_{max}$ [ng/ml] | $T_{max}$ [h] |

Table (continued)

| Bioavailability of Ciclosporin in Humans | | | |
|---|---|---|---|
| Mean (±SD) values of $AUC_{(0-48\ h)}$, $C_{max}$ and $T_{max}$ after single oral administration of different dosages of Composition X and Composition 8 | | | |
| 200 mg Comp X | 2028 ± 608 | 558 ± 228 | 2.1 ± 0.7 |
| 200 mg Comp 8 | 3468 ± 1000 | 1025 ± 218 | 1.5 ± 0.4 |
| 400 mg Comp X | 3326 ± 1115 | 785 ± 252 | 2.1 ± 0.9 |
| 400 mg Comp 8 | 6944 ± 1468 | 1557 ± 286 | 1.4 ± 0.4 |
| 600 mg Comp X | 4501 ± 1217 | 917 ± 236 | 2.3 ± 1.0 |
| 600 mg Comp 8 | 9689 ± 2282 | 1812 ± 400 | 1.7 ± 0.6 |
| 800 mg Comp X | 5209 ± 1554 | 1045 ± 264 | 2.4 ± 1.0 |
| 800 mg Comp 8 | 12162 ± 3059 | 2143 ± 576 | 2.1 ± 0.8 |

Based on the mean ratios of $AUC_{(0-48\ h)}$-values the relative bioavailability of Composition 8 vs Composition X was estimated between 170% and 233%, depending on the dose administered (see following table).

Table

| Relative bioavailability of Composition 8 vs. Composition X | | |
|---|---|---|
| Dose of [mg] | Mean ratio of $AUC_{(0-48\ h)}$ Comp 8 vs. Comp X | Conversion Factor: Comp X vs. Comp 8 |
| 200 | 1.70 | 0.59 |
| 400 | 2.09 | 0.48 |
| 600 | 2.15 | 0.47 |
| 800 | 2.33 | 0.43 |

Conclusion: The composition according to the present invention (Composition 8) has a significantly higher bioavailability in humans be at least factor 1.7 when compared to the commercial form (Composition X).

The accompanying Figure III provides a graphical plot of the mean $AUC_{(0-48\ h)}$ - values of composition X (open triangles) versus those of Composition 8 (solid Circles). AUC-values (in ng.h/ml) of Ciclosporin vertically and dose horizontally as obtained from Example 4.

The extent of absorption of Composition 8 (in terms of $AUC_{(0-48\ h)}$-values) seemed to be independent of dose, whereas the extent of absorption of Composition X declined with increasing doses (see Figure III).

## Claims

### Claims for the following Contracting States : BE, DK, NL, PT, SE

1. A pharmaceutical composition comprising a cyclosporin as active ingredient in a carrier medium comprising:

   1) 1,2-propylene glycol
   2) a mixed $C_{12}$-$C_{20}$ fatty acid mono-, di-, tri-glyceride, and
   3) a surfactant,

   which composition is a microemulsion preconcentrate.

2. A composition according to claim 1, wherein component (2) comprises linolenic, linoleic and oleic acids.

3. A composition according to claim 1 or 2, wherein component (2) comprises a trans-esterification product of a vegetable oil.

4. A composition according to claim 3, wherein the vegetable oil is corn oil.

5. A composition according to claim 3 or 4, wherein the vegetable oil is transesterified with glycerol.

6. A composition according to any one of claims 1 or 5, wherein the free glycerol content is below 1%.

7. A composition according to claim 5 or 6, wherein component (2) comprises Maisine.

8. A composition according to claim 5 or 6 wherein component (2) comprises a refined glycerol-transesterified corn oil.

9. A composition according to claim 8 wherein component (2) comprises a trans-esterification product of corn oil and glycerol comprising predominately of linoleic acid and oleic acid mono-, di- and tri-glycerides treated to enhance the unsaturated fatty acid component content of mono-, di- and tri-glycerides so that the linoleic acid and oleic acid mono-, di- and tri-glyceride content is in total 85% or more of the whole composition.

10. A composition according to any preceding claim wherein component (2) comprises from about 30% to about 40% mono-glycerides, from about 45% to about 55% of di-glycerides and at least 5% of tri-glycerides by weight based on the total weight of component (2).

11. A composition according to claim 10 wherein component (2) comprises from about 32% to about 36% of mono-glycerides, from about 45% to about 55% of di-glycerides, and from about 12% to about 20% of tri-glycerides by weight based on the total weight of component (2).

12. A composition according to any preceding claim, wherein component (3) has an HLB value of at least 10.

13. A composition according to any one of claims 1 to 12, wherein component (3) comprises a reaction product of a natural or hydrogenated castor oil and ethylene oxide.

14. A composition according to any preceding claim, wherein (3) comprises Cremophor RH40.

15. A composition according to any preceding claim wherein the carrier medium additionally comprises a hydrophilic phase co-component.

16. A composition according to claim 15 wherein the hydrophilic phase co-component is ethanol.

17. A composition according to any preceding claim in unit dosage form.

18. A composition according to claim 17, comprising a gelatine encapsulated form.

19. A composition according to any preceding claim additionally comprising water or an aqueous phase and which is a microemulsion.

20. A composition according to any preceding claim, wherein component (1) plus any hydrophilic phase co-component present comprises from 10 to 35% by weight of the hydrophilic phase component (1) plus components (2) and (3).

21. A composition according to claim 22, wherein the hydrophilic phase co-component is present in an amount of from about 25 to 75% of the total weight of component (3) and the hydrophilic phase co-component.

22. A composition according to any preceding claim, wherein the component (2) is present in an amount of from 20 to 40% based on the total weight of hydrophilic phase component(s) plus component (2) and (3).

23. A composition according to any preceding claim, wherein the component (3) is present in an amount of from 30 to 60% based on the total weight of hydrophilic phase component(s) plus component (2) and (3).

24. A composition according to any preceding claim containing from 7.5 to 15% of cyclosporin.

25. A composition according to any preceding claim containing Ciclosporin as the cyclosporin.

13

26. A composition according to any one of claims 1 to 24 containing [O-(2-hydroxyethyl)-(D)Ser][8]-Ciclosporin as the cyclosporin.

27. A composition according to any one of claims 1 to 24 containing [3'-deshydroxy-3'-keto-MeBmt][1]-[Val][2]-Ciclosporin as the cyclosporin.

28. A composition according to any one of claims 1 to 24 containing Cyclosporin G as the ciclosporin.

**Claims for the following Contracting States : ES, GR**

1. A process for the preparation of a pharmaceutical composition comprising a cyclosporin as active ingredient in a carrier medium comprising:

   1) 1,2-propylene glycol
   2) a mixed $C_{12}$-$C_{20}$ fatty acid mono-, di-, tri-glyceride, and
   3) a surfactant, in the form of a microemulsion preconcentrate

   which process comprises bringing a component (1), a component (2) and a component (3) into intimate admixture and, when required compounding the obtained composition in unit dosage form.

2. A process according to claim 1, wherein component (2) comprises linolenic, linoleic and oleic acids.

3. A process according to claim 1 or 2, wherein component (2) comprises a trans-esterification product of a vegetable oil.

4. A process according to claim 3, wherein the vegetable oil is corn oil.

5. A process according to claim 3 or 4, wherein the vegetable oil is transesterified with glycerol.

6. A process according to any one of claims 1 or 5, wherein the free glycerol content is below 1%.

7. A process according to claim 5 or 6, wherein component (2) comprises Maisine.

8. A process according to claim 5 or 6 wherein component (2) comprises a "refined glycerol-transesterified corn oil".

9. A process according to claim 8 wherein component (2) comprises a composition according to any preceding claim which is a trans-esterification product of corn oil and glycerol comprising predominately of linoleic acid and oleic acid mono-, di- and tri-glycerides treated to enhance the unsaturated fatty acid component content of mono-, di- and tri-glycerides so that the linoleic acid and oleic acid mono-, di- and tri-glyceride content is in total 85% or more of the whole composition.

10. A process according to any preceding claim wherein component (2) comprises from about 30% to about 40% mono-glycerides, from about 45% to about 55% of di-glycerides and at least 5% of tri-glycerides by weight based on the total weight of component (2).

11. A process according to claim 10 wherein component (2) comprises from about 32% to about 36% of mono-glycerides, from about 45% to about 55% of di-glycerides, and from about 12% to about 20% of tri-glycerides by weight based on the total weight of component (2).

12. A process according to any preceding claim, wherein component (3) has an HLB value of at least 10.

13. A process according to any one of claims 1 to 12, wherein component (3) comprises a reaction product of a natural or hydrogenated castor oil and ethylene oxide.

14. A process according to any preceding claim, wherein (3) comprises Cremophor RH40.

15. A process according to any preceding claim wherein the carrier medium additionally comprises a hydrophilic phase co-component.

**16.** A process according to claim 15 wherein the hydrophilic phase co-component is ethanol.

**17.** A process according to any preceding claim in unit dosage form.

**18.** A process according to claim 17, comprising a gelatine encapsulated form.

**19.** A process according to any preceding claim additionally comprising water or an aqueous phase and which is a microemulsion.

**20.** A process according to any preceding claim, wherein component (1) plus any hydrophilic phase co-component present comprises from 10 to 35% by weight of the hydrophilic phase component (1) plus components (2) and (3).

**21.** A process according to claim 20, wherein the hydrophilic phase co-component is present in an amount of from about 25 to 75% of the total weight of component (3) and the hydrophilic phase co-component.

**22.** A process according to any preceding claim, wherein the component (2) is present in an amount of from 20 to 40% based on the total weight of hydrophilic phase component(s) plus component (2) and (3).

**23.** A process according to any preceding claim, wherein the component (3) is present in an amount of from 30 to 60% based on the total weight of hydrophilic phase component(s) plus component (2) and (3).

**24.** A process according to any preceding claim containing from 7.5 to 15% of cyclosporin.

**25.** A process according to any preceding claim containing Ciclosporin as the cyclosporin.

**26.** A process according to any one of claims 1 to 24 containing [O-(2-hydroxyethyl)-(D)Ser]$^8$-Ciclosporin as the cyclosporin.

**27.** A process according to any one of claims 1 to 24 containing [3'-deshydroxy-3'-keto-MeBmt]$^1$-[Val]$^2$-Ciclosporin as the cyclosporin.

**28.** A process according to any one of claims 1 to 24 containing Cyclosporin G as the ciclosporin.

**29.** A pharmaceutical composition comprising a cyclosporin as active ingredient in a carrier medium comprising:

> 1) 1,2-propylene glycol
> 2) a mixed $C_{12}$-$C_{20}$ fatty acid mono-, di-, tri-glyceride, and
> 3) a surfactant, which composition is a microemulsion preconcentrate.

**30.** A composition as claimed in claim 29 wherein

> the cyclosporin is cyclosporin A;

> component (2) comprises from about 30% to about 40% mono-glycerides, from about 45% to about 55% of di-glycerides and at least 5% of triglycerides by weight based on the total weight of component (2) ;

> component (3) comprises Cremophor RH40; and

> the carrier medium additionally comprises ethanol.

**Patentansprüche**

**Patentansprüche für folgende Vertragsstaaten : BE, DK, NL, PT, SE**

**1.** Pharmazeutische Zusammensetzung, enthaltend ein Cyclosporin als Wirkstoff in einem Trägermedium, das enthält

> (1) 1,2-Propylenglycol,

(2) ein Gemisch von $C_{12}$-$C_{20}$-Fettsäuremono-, -di-, -triglycerid und

(3) ein oberflächenaktives Mittel,

wobei diese Zusammensetzung ein Mikroemulsionsvorkonzentrat ist.

2. Zusammensetzung nach Anspruch 1, worin die Komponente (2) Linolensäure, Linolsäure und Ölsäure enthält.

3. Zusammensetzung nach Anspruch 1 oder 2, worin die Komponente (2) ein Umesterungsprodukt eines Pflanzenöls enthält.

4. Zusammensetzung nach Anspruch 3, worin das Pflanzenöl Maisöl ist.

5. Zusammensetzung nach Anspruch 3 oder 4, worin das Pflanzenöl mit Glycerin umgeestert ist.

6. Zusammensetzung nach einem der Ansprüche 1 oder 5, worin der Gehalt an freiem Glycerin unter 1% liegt.

7. Zusammensetzung nach Anspruch 5 oder 6, worin die Komponente (2) Maisine enthält.

8. Zusammensetzung nach Anspruch 5 oder 6, worin die Komponente (2) ein raffiniertes Glycerin-umgeestertes Maisöl enthält.

9. Zusammensetzung nach Anspruch 8, worin die Komponente (2) ein Umesterungsprodukt von Maisöl und Glycerin ist, das vorwiegend Linolsäure- und Ölsäuremono-, -di- und -triglyceride enthält, welches so behandelt worden ist, daß die ungesättigte Fettsäurekomponente der Mono-, -Di- und Triglyceride in der Weise angereichert wird, daß der Gehalt an Linolsäure- und Ölsäuremono-, -di- und -triglyceriden insgesamt 85% oder mehr der gesamten Zusammensetzung beträgt.

10. Zusammensetzung nach einem der vorhergehenden Ansprüche, worin die Komponente (2) etwa 30 Gew.-% bis etwa 40 Gew.-% Monoglyceride, etwa 45 Gew.-% bis etwa 55 Gew.-% Diglyceride und wenigstens 5 Gew.-% Triglyceride, bezogen auf das Gesamtgewicht der Komponente (2), enthält.

11. Zusammensetzung nach Anspruch 10, worin die Komponente (2) etwa 32 Gew.-% bis etwa 36 Gew.-% Monoglyceride, etwa 45 Gew.-% bis etwa 55 Gew.-% Diglyceride und etwa 12 Gew.-% bis etwa 20 Gew.-% Triglyceride, bezogen auf das Gesamtgewicht der Komponente (2), enthält.

12. Zusammensetzung nach einem der vorhergehenden Ansprüche, worin die Komponente (3) einen HLB-Wert von wenigstens 10 hat.

13. Zusammensetzung nach einem der Ansprüche 1 bis 12, worin die Komponente (3) ein Reaktionsprodukt aus einem natürlichen oder hydrierten Ricinusöl und Ethylenoxid enthält.

14. Zusammensetzung nach einem der vorhergehenden Ansprüche, worin die Komponente (3) Cremophor RH 40 enthält.

15. Zusammensetzung nach einem der vorhergehenden Ansprüche, worin das Trägermedium zusätzlich eine Cokomponente der hydrophilen Phase enthält.

16. Zusammensetzung nach Anspruch 15, worin die Cokomponente der hydrophilen Phase Ethanol ist.

17. Zusammensetzung nach einem der vorhergehenden Ansprüche als Einheitsdosierungsform.

18. Zusammensetzung nach Anspruch 17 als eine in Gelatine verkapselte Form.

19. Zusammensetzung nach einem der vorhergehenden Ansprüche, welche zusätzlich Wasser oder eine wäßrige Phase enthält und eine Mikroemulsion darstellt.

20. Zusammensetzung nach einem der vorhergehenden Ansprüche, worin die Komponente (1) zusammen mit jeder vorhandenen Cokomponente der hydrophilen Phase 10 bis 35 Gew.-% beträgt, bezogen auf das Gewicht der hydrophilen Phase gemäß Komponente (1) und den Komponenten (2) und (3).

21. Zusammensetzung nach Anspruch 20, worin die Cokomponente der hydrophilen Phase in einer Menge von etwa 25 bis 75 Gew.-% vorhanden ist, bezogen auf das Gesamtgewicht der Komponente (3) und der Cokomponente der hydrophilen Phase.

22. Zusammensetzung nach einem der vorhergehenden Ansprüche, worin die Komponente (2) in einer Menge von 20 bis 40 Gew.-% vorhanden ist, bezogen auf das Gesamtgewicht der Komponente(n) der hydrophilen Phase und den Komponenten (2) und (3).

23. Zusammensetzung nach einem der vorhergehenden Ansprüche, worin die Komponente (3) in einer Menge von 30 bis 60 Gew.-% vorhanden ist, bezogen auf das Gesamtgewicht der Komponente(n) der hydrophilen Phase und den Komponenten (2) und (3).

24. Zusammensetzung nach einem der vorhergehenden Ansprüche, enthaltend 7,5 bis 15% Cyclosporin.

25. Zusammensetzung nach einem der vorhergehenden Ansprüche, enthaltend Ciclosporin als das Cyclosporin.

26. Zusammensetzung nach einem der Ansprüche 1 bis 24, enthaltend [O-(2-Hydroxyethyl)-(D)Ser]$^8$-Ciclosporin als das Cyclosporin.

27. Zusammensetzung nach einem der Ansprüche 1 bis 24, enthaltend [3'-Dehydroxy-3'-keto-MeBmt]$^1$-[Val]$^2$-Ciclosporin als das Cyclosporin.

28. Zusammensetzung nach einem der Ansprüche 1 bis 24, enthaltend Cyclosporin G als das Ciclosporin.

**Patentansprüche für folgende Vertragsstaaten : ES, GR**

1. Verfahren zur Herstellung einer pharmazeutischen Zusammensetzung, die ein Cyclosporin als Wirkstoff in einem Trägermedium enthält, enthaltend

   (1) 1,2-Propylenglycol,
   (2) ein Gemisch von $C_{12}$-$C_{20}$-Fettsäuremono-, -di-, -triglycerid und
   (3) ein oberflächenaktives Mittel,

   wobei eine Komponente (1), eine Komponente (2) und eine Komponente (3) zu einem innigen Gemisch verarbeitet wird und, falls erforderlich, die erhaltene Zusammensetzung in eine Einheitsdosierungsform gebracht wird.

2. Verfahren nach Anspruch 1, worin die Komponente (2) Linolensäure, Linolsäure und Ölsäure enthält.

3. Verfahren nach Anspruch 1 oder 2, worin die Komponente (2) ein Umesterungsprodukt eines Pflanzenöls enthält.

4. Verfahren nach Anspruch 3, worin das Pflanzenöl Maisöl ist.

5. Verfahren nach Anspruch 3 oder 4, worin das Pflanzenöl mit Glycerin umgeestert ist.

6. Verfahren nach einem der Ansprüche 1 oder 5, worin der Gehalt an freiem Glycerin unter 1% liegt.

7. Verfahren nach Anspruch 5 oder 6, worin die Komponente (2) Maisine enthält.

8. Verfahren nach Anspruch 5 oder 6, worin die Komponente (2) ein raffiniertes Glycerin-umgeestertes Maisöl enthält.

9. Verfahren nach Anspruch 8, worin die Komponente (2) eine Zusammensetzung nach einem der vorhergehenden Ansprüche enthält, die ein Umesterungsprodukt von Maisöl und Glycerin ist, das vorwiegend Linolsäure- und Ölsäuremono-, -di- und -triglyceride enthält, welches so behandelt worden ist, daß die ungesättigte Fettsäurekomponente der Mono-, -Di- und Triglyceride in der Weise angereichert wird, daß der Gehalt an Linolsäure- und Ölsäuremono-, -di- und -triglyceriden insgesamt 85% oder mehr der gesamten Zusammensetzung beträgt.

**10.** Verfahren nach einem der vorhergehenden Ansprüche, worin die Komponente (2) etwa 30 Gew.-% bis etwa 40 Gew.-% Monoglyceride, etwa 45 Gew.-% bis etwa 55 Gew.-% Diglyceride und wenigstens 5 Gew.-% Triglyceride, bezogen auf das Gesamtgewicht der Komponente (2), enthält.

**11.** Verfahren nach Anspruch 10, worin die Komponente (2) etwa 32 Gew.-% bis etwa 36 Gew.-% Monoglyceride, etwa 45 Gew.-% bis etwa 55 Gew.-% Diglyceride und etwa 12 Gew.-% bis etwa 20 Gew.-% Triglyceride, bezogen auf das Gesamtgewicht der Komponente (2), enthält.

**12.** Verfahren nach einem der vorhergehenden Ansprüche, worin die Komponente (3) einen HLB-Wert von wenigstens 10 hat.

**13.** Verfahren nach einem der Ansprüche 1 bis 12, worin die Komponente (3) ein Reaktionsprodukt aus einem natürlichen oder hydrierten Ricinusöl und Ethylenoxid enthält.

**14.** Verfahren nach einem der vorhergehenden Ansprüche, worin die Komponente (3) Cremophor RH 40 enthält.

**15.** Verfahren nach einem der vorhergehenden Ansprüche, worin das Trägermedium zusätzlich eine Cokomponente der hydrophilen Phase enthält.

**16.** Verfahren nach Anspruch 15, worin die Cokomponente der hydrophilen Phase Ethanol ist.

**17.** Verfahren nach einem der vorhergehenden Ansprüche als Einheitsdosierungsform.

**18.** Verfahren nach Anspruch 17 als eine in Gelatine verkapselte Form.

**19.** Verfahren nach einem der vorhergehenden Ansprüche, welches zusätzlich Wasser oder eine wäßrige Phase enthält und eine Mikroemulsion darstellt.

**20.** Verfahren nach einem der vorhergehenden Ansprüche, worin die Komponente (1) zusammen mit jeder vorhandenen Cokomponente der hydrophilen Phase 10 bis 35 Gew.-% beträgt, bezogen auf das Gewicht der hydrophilen Phase gemäß Komponente (1) und den Komponenten (2) und (3).

**21.** Verfahren nach Anspruch 20, worin die Cokomponente der hydrophilen Phase in einer Menge von etwa 25 bis 75 Gew.-% vorhanden ist, bezogen auf das Gesamtgewicht der Komponente (3) und der Cokomponente der hydrophilen Phase.

**22.** Verfahren nach einem der vorhergehenden Ansprüche, worin die Komponente (2) in einer Menge von 20 bis 40 Gew.-% vorhanden ist, bezogen auf das Gesamtgewicht der Komponente(n) der hydrophilen Phase und den Komponenten (2) und (3).

**23.** Verfahren nach einem der vorhergehenden Ansprüche, worin die Komponente (3) in einer Menge von 30 bis 60 Gew.-% vorhanden ist, bezogen auf das Gesamtgewicht der Komponente(n) der hydrophilen Phase und den Komponenten (2) und (3).

**24.** Verfahren nach einem der vorhergehenden Ansprüche, enthaltend 7,5 bis 15% Cyclosporin.

**25.** Verfahren nach einem der vorhergehenden Ansprüche, enthaltend Ciclosporin als das Cyclosporin.

**26.** Verfahren nach einem der Ansprüche 1 bis 24, enthaltend [O-(2-Hydroxyethyl)-(D)Ser][8]-Ciclosporin als das Cyclosporin.

**27.** Verfahren nach einem der Ansprüche 1 bis 24, enthaltend [3'-Dehydroxy-3'-keto-MeBmt][1]-[Val][2]-Ciclosporin als das Cyclosporin.

**28.** Verfahren nach einem der Ansprüche 1 bis 24, enthaltend Cyclosporin G als das Ciclosporin.

**29.** Pharmazeutische Zusammensetzung, die ein Cyclosporin als Wirkstoff in einem Trägermedium enthält, enthaltend

(1) 1,2-Propylenglycol,
(2) ein Gemisch von $C_{12}$-$C_{20}$-Fettsäuremono-, -di-, -triglycerid und
(3) ein oberflächenaktives Mittel,

wobei diese Zusammensetzung ein Mikroemulsionsvorkonzentrat ist.

30. Zusammensetzung nach Anspruch 29, worin

das Cyclosporin Cyclosporin A ist,
die Komponente (2) etwa 30 Gew.-% bis etwa 40 Gew.-% Monoglyceride, etwa 45 Gew.-% bis etwa 55 Gew.-% Diglyceride und
wenigstens 5 Gew.-% Triglyceride, bezogen auf das Gesamtgewicht der Komponente (2), enthält,
die Komponente (3) Cremophor RH 40 enthält, und das Trägermedium zusätzlich Ethanol enthält.

**Revendications**

**Revendications pour les Etats contractants suivants : BE, DK, NL, PT, SE**

1. Une composition pharmaceutique, caractérisée en ce qu'elle comprend une cyclosporine comme substance active, dans un véhicule constitué par :

   1) du 1,2-propylèneglycol
   2) un mélange de mono-, di-, tri-glycérides d'acides gras en $C_{12}$-$C_{20}$, et
   3) un tensio-actif,

   ladite composition étant un pré-concentré de micro-émulsion.

2. Une composition selon la revendication 1, caractérisée en ce que le composant (2) comprend l'acide linolénique, l'acide linoléique et l'acide oléique.

3. Une composition selon la revendication 1 ou 2, caractérisée en ce que le composant (2) comprend un produit de transestérification d'une huile végétale.

4. Une composition selon la revendication 3, caractérisée en ce que l'huile végétale est l'huile de maïs.

5. Une composition selon la revendication 3 ou 4, caractérisée en ce que l'huile végétale est transestérifiée avec du glycérol.

6. Une composition selon l'une quelconque des revendications 1 ou 5, caractérisée en ce que la teneur en glycérol libre est inférieure à 1%.

7. Une composition selon la revendication 5 ou 6, caractérisée en ce que le composant (2) comprend de la Maisine.

8. Une composition selon la revendication 5 ou 6, caractérisée en ce que le composant (2) comprend de l'huile de maïs transestérifiée avec du glycérol et raffinée.

9. Une composition selon la revendication 8, caractérisée en ce que le composant (2) est le produit de transestérification de l'huile de mais avec le glycérol comprenant de façon prédominante des mono-, di- et triglycérides de l'acide linoléique, et de l'acide oléique, traité pour augmenter la teneur en composant acides gras insaturés des mono-, di- et triglycérides, de sorte que la teneur en mono-, di- et triglycérides de l'acide linoléique et de l'acide oléique représente au total 85% ou plus du total de la composition.

10. Une composition selon l'une quelconque des revendications précédentes, caractérisée en ce que le composant (2) comprend d'environ 30% à environ 40% de monoglycérides, d'environ 45% à environ 55% de diglycérides et au moins 5% de triglycérides, en poids par rapport au poids total du composant (2).

11. Une composition selon la revendication 10, caractérisée en ce que le composant (2) comprend d'environ 32% à environ 36% de monoglycérides, d'environ 45% à environ 55% de diglycérides et d'environ 12% à environ 20% de tri-glycérides, en poids par rapport au poids total du composant (2).

12. Une composition selon l'une quelconque des revendications précédentes, caractérisée en ce que le composant (3) a une valeur HLB d'au moins 10.

13. Une composition selon l'une quelconque des revendications 1 à 12, caractérisée en ce que le composant (3) comprend un produit de réaction d'une huile de ricin naturelle ou hydrogénée avec de l'oxyde d'éthylène.

14. Une composition selon l'une quelconque des revendications précédentes, caractérisée en ce que (3) comprend le Cremophor RH40.

15. Une composition selon l'une quelconque des revendications précédentes, caractérisée en ce que le véhicule comprend également un co-composant de la phase hydrophile.

16. Une composition selon la revendication 15, caractérisée en ce que le co-composant de la phase hydrophile est l'éthanol.

17. Une composition selon l'une quelconque des revendications précédentes, sous forme de doses unitaires.

18. Une composition selon la revendication 17, caractérisée en ce qu'elle se présente sous forme de capsules de gélatine dures ou molles.

19. Une composition selon l'une quelconque des revendications précédentes, caractérisée en ce qu'elle comprend également de l'eau ou une phase aqueuse et se présente sous forme d'une micro-émulsion.

20. Une composition selon l'une quelconque des revendications précédentes, caractérisée en ce que le composant (1) plus tout co-composant de la phase hydrophile présent, représente de 10 à 35% en poids du poids total du composant (1) de la phase hydrophile plus les composants (2) et (3).

21. Une composition selon la revendication 20, caractérisée en ce que le co-composant de la phase hydrophile est présent en une quantité comprise entre environ 25 et 75% du poids total du composant (3) et du co-composant de la phase hydrophile.

22. Une composition selon l'une quelconque des revendications précédentes, caractérisée en ce que le composant (2) est présent en une quantité comprise entre 20 à 40% par rapport au poids total du ou des composants de la phase hydrophile plus les composants (2) et (3).

23. Une composition selon l'une quelconque des revendications précédentes, caractérisée en ce que le composant (3) est présent en une quantité comprise entre 30 à 60% par rapport au poids total du ou des composants de la phase hydrophile plus les composants (2) et (3).

24. Une composition selon l'une quelconque des revendications précédentes, caractérisée en ce qu'elle contient de 7,5 à 15% de cyclosporine.

25. Une composition selon l'une quelconque des revendications précédentes, caractérisée en ce qu'elle contient de la Ciclosporine comme cyclosporine.

26. Une composition selon l'une quelconque des revendications 1 à 24, caractérisée en ce qu'elle contient la [0-(2-hydroxyéthyl)-(D)Ser][8]-Ciclosporine comme cyclosporine.

27. Une composition selon l'une quelconque des revendications 1 à 24, caractérisée en ce qu'elle contient la [3'-deshydroxy-3'-céto-MeBmt][1]-[Val][2]-Ciclosporine comme cyclosporine.

28. Une composition selon l'une quelconque des revendications 1 à 24, caractérisée en ce qu'elle contient la Cyclosporine G comme ciclosporine.

**Revendications pour les Etats contractants suivants : ES, GR**

1. Un procédé de préparation d'une composition pharmaceutique comprenant une cyclosporine comme substance active, dans un véhicule constitué par:

   1) du 1,2-propylèneglycol
   2) un mélange de mono-, di-, tri-glycérides d'acides gras en $C_{12}$-$C_{20}$, et
   3) un tensio-actif, sous forme d'un pré-concentré de micro-émulsion,

   procédé caractérisé en ce qu'on mélange à fond un composant (1), un composant (2) et un composant (3) et, si nécessaire, on met la composition obtenue sous forme de doses unitaires.

2. Un procédé selon la revendication 1, caractérisé en ce que le composant (2) comprend l'acide linolénique, l'acide linoléique et l'acide oléique.

3. Un procédé selon la revendication 1 ou 2, caractérisé en ce que le composant (2) comprend un produit de transestérification d'une huile végétale.

4. Un procédé selon la revendication 3, caractérisé en ce que l'huile végétale est l'huile de maïs.

5. Un procédé selon la revendication 3 ou 4, caractérisé en ce que l'huile végétale est transestérifiée avec du glycérol.

6. Un procédé selon l'une quelconque des revendications 1 ou 5, caractérisé en ce que la teneur en glycérol libre est inférieure à 1%.

7. Un procédé selon la revendication 5 ou 6, caractérisé en ce que le composant (2) comprend de la Maisine.

8. Un procédé selon la revendication 5 ou 6, caractérisé en ce que le composant (2) comprend "de l'huile de maïs transestérifiée avec du glycérol et raffinée".

9. Un procédé selon la revendication 8, caractérisé en ce que le composant (2) comprend une composition selon l'une quelconque des revendications précédentes qui est un produit de transestérification de l'huile de maïs avec le glycérol comprenant de façon prédominante des mono-, di- et triglycérides de l'acide linoléique et de l'acide oléique, traité pour augmenter la teneur en composant acides gras insaturés des mono-, di- et triglycérides, de sorte que la teneur en mono-, di- et triglycérides de l'acide linoléique et de l'acide oléique représente au total 85% ou plus total de la composition.

10. Un procédé selon l'une quelconque des revendications précédentes, caractérisé en ce que le composant (2) comprend d'environ 30% à environ 40% de monoglycérides, d'environ 45% à environ 55% de diglycérides et au moins 5% de triglycérides, en poids par rapport au poids total du composant (2).

11. Un procédé selon la revendication 10, caractérisé en ce que le composant (2) comprend d'environ 32% à environ 36% de monoglycérides, d'environ 45% à environ 55% de diglycérides et d'environ 12% à environ 20% de triglycérides, en poids par rapport au poids total du composant (2).

12. Un procédé selon l'une quelconque des revendications précédentes, caractérisé en ce que le composant (3) a une valeur HLB d'au moins 10.

13. Un procédé selon l'une quelconque des revendications 1 à 12, caractérisé en ce que le composant (3) comprend un produit de réaction d'une huile de ricin naturelle ou hydrogénée avec de l'oxyde d'éthylène.

14. Un procédé selon l'une quelconque des revendications précédentes, caractérisé en ce que (3) comprend le Cremophor RH40.

15. Un procédé selon l'une quelconque des revendications précédentes, caractérisé en ce que le véhicule comprend également un co-composant de la phase hydrophile.

16. Un procédé selon la revendication 15, caractérisé en ce que le co-composant de la phase hydrophile est l'éthanol.

**17.** Un procédé selon l'une quelconque des revendications précédentes, sous forme de doses unitaires.

**18.** Un procédé selon la revendication 17, comprenant une forme de capsules de gélatine dures ou molles.

**19.** Un procédé selon l'une quelconque des revendications précédentes, comprenant également de l'eau ou une phase aqueuse et se présentant sous forme d'une micro-émulsion.

**20.** Un procédé selon l'une quelconque des revendications précédentes, caractérisé en ce que le composant (1) plus tout co-composant de la phase hydrophile présent, représente de 10 à 35% en poids du composant (1) de la phase hydrophile plus les composants (2) et (3).

**21.** Un procédé selon la revendication 20, caractérisé en ce que le co-composant de la phase hydrophile est présent en une quantité comprise entre environ 25 et 75% du poids total du composant (3) et du co-composant de la phase hydrophile.

**22.** Un procédé selon l'une quelconque des revendications précédentes, caractérisé en ce que le composant (2) est présent en une quantité comprise entre 20 à 40% par rapport au poids total du ou des composants de la phase hydrophile plus les composants (2) et (3).

**23.** Un procédé selon l'une quelconque des revendications précédentes, caractérisé en ce que le composant (3) est présent en une quantité comprise entre 30 à 60% par rapport au poids total du ou des composants de la phase hydrophile plus les composants (2) et (3).

**24.** Un procédé selon l'une quelconque des revendications précédentes, contenant de 7,5 à 15% de cyclosporine.

**25.** Un procédé selon l'une quelconque des revendications précédentes, contenant de la Ciclosporine comme cyclosporine.

**26.** Un procédé selon l'une quelconque des revendications 1 à 24, contenant la [0-(2-hydroxyéthyl)-(D)Ser]$^8$-Ciclosporine comme cyclosporine.

**27.** Un procédé selon l'une quelconque des revendications 1 à 24, contenant la [3'-deshydroxy-3'-céto-MeBmt]$^1$-[Val]$^2$-Ciclosporine comme cyclosporine.

**28.** Un procédé selon l'une quelconque des revendications 1 à 24, contenant la Cyclosporine G comme ciclosporine.

**29.** Une composition pharmaceutique, caractérisée en ce qu'elle comprend une cyclosporine comme substance active, dans un véhicule constitué par:

    1) du 1,2-propylèneglycol
    2) un mélange de mono-, di-, tri-glycérides d'acides gras en $C_{12}$-$C_{20}$, et
    3) un tensio-actif,

ladite composition étant un pré-concentré de micro-émulsion.

**30.** Une composition telle que spécifiée à la revendication 29, caractérisée en ce que

    la cyclosporine est la cyclosporine A;

    le composant (2) comprend entre environ 30% et environ 40% de mono-glycérides, entre environ 45% et environ 55% de di-glycérides et au moins 5% de triglycérides en poids par rapport au poids total du composant (2);

    le composant (3) comprend du Cremophor RH40; et

    le véhicule comprend en outre de l'éthanol.

Fig. I

Fig. II

Legend:
- ○ Composition X
- ● Composition I
- ⊢⊣ Standard deviation

Figure: Blood conc. [ng/ml] versus Time [h]

Fig. III

Mean AUC(0-48h)-Values of Ciclosporin After Single
Oral Doses of Composition X and of Composition 8

Dose (mg)

—●— Composition 8
—△— Composition X

EP 0 539 319 B1